(19) Europäisches Patentamt
European Patent Office
Office européen des brevets

(11) Veröffentlichungsnummer: **0 234 517 A1**

(12) # EUROPÄISCHE PATENTANMELDUNG

(21) Anmeldenummer: **87102399.0**

(22) Anmeldetag: **20.02.87**

(51) Int. Cl.[3]: **C 07 D 471/04**
**A 61 K 31/435**
**//(C07D471/04, 221:00, 209:00)**

(30) Priorität: **22.02.86 DE 3605742**

(43) Veröffentlichungstag der Anmeldung:
**02.09.87 Patentblatt 87/36**

(84) Benannte Vertragsstaaten:
**AT BE CH DE ES FR GB GR IT LI LU NL SE**

(71) Anmelder: **GÖDECKE AKTIENGESELLSCHAFT**
**Salzufer 16**
**D-1000 Berlin 10(DE)**

(72) Erfinder: **Mannhardt, Karl, Dr.**
**Pfauenstrasse 14**
**D-7807 Elzach-Oberprechtal(DE)**

(72) Erfinder: **Klimars, Michael, Dr.**
**Kandelstrasse 14**
**D-7803 Gundelfingen(DE)**

(72) Erfinder: **Hartenstein, Johannes, Dr.**
**Fohrenbühl 23**
**D-7801 Stegen-Wittenhal(DE)**

(72) Erfinder: **Wagner, Bernd, Dr.**
**Am Lossele 5**
**D-7809 Denzlingen(DE)**

(72) Erfinder: **Weinheimer, Günter, Dr.**
**Sachenstrasse 4**
**D-7809 Denzlingen(DE)**

(72) Erfinder: **Steinbrecher, Wolfgang, Dr.**
**Schwarzwaldstrasse 16**
**D-7803 Gundelfingen(DE)**

(54) **Pyrrolo ]3,4-b[ pyridine, Verfahren zu deren Herstellung und diese enthaltende Arzneimittel zur Behandlung von Gefässerkrankungen.**

(57) Es werden neue Pyrrolo[3,4-b]pyridin-Derivate der allgemeinen Formel I

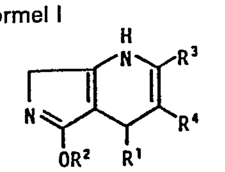

(I)

bzw. für den Fall, daß $R^2$ Wasserstoff bedeutet, deren tautomere Form der allgemeinen Formel Ia

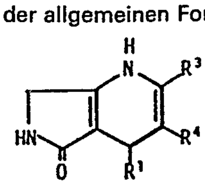

(Ia)

beschrieben,
in welchen

$R^1$ einen unsubstituierten oder substituierten aromatischen oder heteroaromatischen Ring oder ein kondensiertes aromatisches oder heterocyclisches Ringsystem bedeutet,

$R^2$ Wasserstoff oder eine Methyl- oder Ethylgruppe darstellt,

$R^3$ einen Kohlenwasserstoffrest mit bis zu 3 Kohlenstoffatomen bedeutet, der auch Sauerstoff-, Schwefel- oder Stickstoffatome enthalten kann,

$R^4$ einen Alkoxycarbonyl- oder einen Carboxamidrest mit bis zu 11 Kohlenstoffatomen, der gegebenenfalls auch Sauerstoff- oder Stickstoffatome enthalten kann, oder eine Carboxylgruppe bedeutet,

sowie gegebenenfalls deren pharmakologisch unbedenkliche Salze.

Weiterhin werden Verfahren zur Herstellung der Verbindungen I bzw. Ia, sowie die Verwendung derselben bei der Bekämpfung von Gefäßkrankheiten beschrieben.

EP 0 234 517 A1

Croydon Printing Company Ltd.

0234517

# BESCHREIBUNG

Die Erfindung betrifft neue Pyrrolo[3,4-b]pyridin-Derivate der allgemeinen Formel I

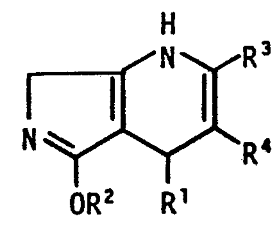

(I)

bzw. für den Fall, daß $R^2$ Wasserstoff bedeutet, deren tautomere Form der allgemeinen Formel Ia

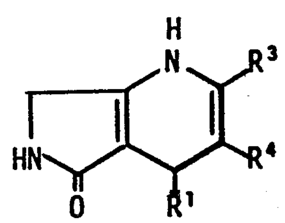

(Ia)

in welchen

$R^1$ einen unsubstituierten oder substituierten aromatischen oder heteroaromatischen Ring oder ein kondensiertes aromatisches oder heterocyclisches Ringsystem bedeutet,

$R^2$ Wasserstoff oder eine Methyl- oder Ethylgruppe darstellt,

$R^3$ einen Kohlenwasserstoffrest mit bis zu 3 Kohlenstoffatomen bedeutet, der auch Sauerstoff-, Schwefel- oder Stickstoffatome enthalten kann,

$R^4$ einen Alkoxycarbonyl- oder einen Carboxamidrest mit bis zu 11 Kohlenstoffatomen, der gegebenenfalls auch Sauerstoff- oder Stickstoffatome enthalten kann, oder eine Carboxylgruppe bedeutet,

sowie gegebenenfalls deren pharmakologisch unbedenkliche Salze.

Bevorzugt werden Pyrrolo[3,4-b]pyridin-Derivate der allgemeinen Formel I

in welcher

$R^1$ einen unsubstituierten oder durch Halogen, Cyano, Nitro, Difluormethoxy oder Trifluormethyl mono- oder disubstituierten Phenylrest, einen Naphthylrest oder einen 2-Pyridyl-, 2-Thienyl- oder 2,1,3-Benzoxadiazolylrest bedeutet,

$R^2$ eine Methylgruppe darstellt,

$R^3$ eine Methyl-, Aminoethoxymethyl- oder Aminoethylthiomethylgruppe bedeutet,

$R^4$ einen Carboxyl- oder einen Alkoxycarbonylrest der allgemeinen Formel II

$$-CO_2R^5 \qquad (II)$$

in welcher $R^5$ entweder Wasserstoff, eine Methyl-, Ethyl- oder Benzylgruppe, eine Aminoalkylgruppe, insbesondere die N-Benzyl-N-Methylaminoethylgruppe bedeutet oder einen Carboxamidrest der allgemeinen Formel III,

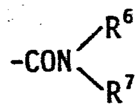

(III)

worin $R^6$ und $R^7$ gleich oder verschieden sein können und Wasserstoff, eine Ethyl- oder N-Benzyl-N-methylaminoethylgruppe bedeuten, darstellt.

Es sind zwar bereits Beispiele für Pyrrolo[3,4-b]pyridine beschrieben, die jedoch im Gegensatz zu den erfindungsgemäßen Verbindungen der allgemeinen Formeln I bzw. Ia am Lactamstickstoffatom substituiert sind (vgl. US 4,284,634); außerdem findet man keine Daten über biologische Wirkungen solcher Verbindungen.

Überraschenderweise wurde nun gefunden, daß die erfindungsgemäßen Verbindungen der allgemeinen Formeln I bzw. Ia wertvolle pharmakologische Eigenschaften besitzen.

Die Erfindung betrifft auch ein Verfahren zur Herstellung von Pyrrolo-[3,4-b]pyridin-Derivaten der allgemeinen Formeln I bzw. Ia dadurch gekennzeichnet, daß man 2-Phthalimidomethyl-1,4-dihydropyridin-Derivate der allgemeinen Formel IV

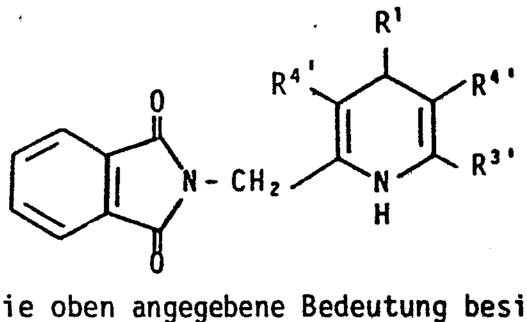

(IV)

in welcher $R^1$ die oben angegebene Bedeutung besitzt, $R^{3'}$ für eine Methylgruppe steht und $R^{4'}$ einen Alkoxycarbonylrest der allgemeinen Formel II bedeutet, mit Hilfe einer Base wie z.B. einem Aminoalkohol, durch Ringschluß in die Pyrrolo[3,4-b]pyridin-Derivate der allgemeinen Formel Ia überführt und anschließend in an sich bekannter Weise am Lactamsauerstoffatom alkyliert.

Verbindungen der allgemeinen Formel I, bei denen $R^3$ eine Aminoethoxymethyl- oder Aminoethylthiomethylgruppe bedeutet, werden hergestellt, indem man Verbindungen der allgemeinen Formel I, bei denen $R^3$ für eine Methylgruppe steht, an dieser bromiert und mit entsprechend substituierten Alkoholen oder Thiolen umsetzt.

Verbindungen der allgemeinen Formel I, bei denen $R^4$ für eine Carboxylgruppe oder eine substituierte oder unsubstituierte Carboxamidgruppe steht, werden hergestellt, indem man Verbindungen der allgemeinen Formel I, bei denen $R^4$ einen Benzyloxycarbonylrest bedeutet, in an sich bekannter Weise hydrogenolytisch (vgl. z.B. Houben-Weyl, Methoden der organischen Chemie, 4. Auflage, Bd. IV/1C, Reduktion Teil 1, 1980, S. 381-382) zur entsprechenden Carbonsäure spaltet und diese wiederum in an sich bekannter Weise über die Zwischenstufe eines Säurehalogenides mit entsprechend substituierten Aminen zur Reaktion bringt.

Die 2-Phthalimidomethyl-1,4-dihydropyridine der allgemeinen Formel IV sind bekannt und können durch Umsetzung von γ-Phthalimidoacetessigsäureestern mit Aminocrotonsäureestern und entsprechenden Aldehyden hergestellt werden (vgl. P 26 58 183).

In der Literatur ist bisher nur die Umsetzung der Phthalimido-Derivate der allgemeinen Formel IV mit Hydrazin zu entsprechenden offenkettigen Aminomethylverbindungen beschrieben (vgl. P 27 40 080). Verwendet man dagegen Ethanolamin als Base, so erhält man überraschenderweise in nahezu quantitativer Ausbeute den Ringschluß zu den Lactamen der allgemeinen Formel Ia. Die Durchführung dieser Reaktion erfolgt bevorzugt in Ethanolamin, das gleichermaßen als Lösungsmittel und Reagens dient. Man arbeitet im Temperaturbereich von 60 - 100°C, bevorzugt jedoch bei 80°C. Die Produkte werden wiederum durch Kristallisation gereinigt.

Die Alkylierung am Lactamsauerstoffatom erfolgt nach üblichen, in der Literatur beschriebenen Verfahren (vgl. Adv. Heterocyclic Chem. 12 (1970), 185-212). Zur selektiven Alkylierung wird bevorzugt Trimethyl- oder Triethyloxoniumtetrafluoroborat in geeigneten Lösungsmitteln wie chlorierten Kohlenwasserstoffen oder cyclischen Ethern, bevorzugt 1,2-Dichlorethan, im Temperaturbereich von -20°C bis 40°C, vorzugsweise jedoch 0°C, eingesetzt.

Die Umsetzung der Brommethyldihydropyridine mit Alkoholen und Thiolen kann im Eintopfverfahren mit Lösungen oder Suspensionen von mindestens drei Äquivalenten eines geeignet substituierten Alkoholates oder Thiolates in einem inerten organischen Lösungsmittel durchgeführt werden.

Dabei werden die Alkoholate bzw. Thiolate vorzugsweise aus dem entsprechenden Alkohol oder Thiol mit einer geeigneten Base, wie z.B. Natriumhydrid, in einem Lösungsmittel wie z.B. einem offenkettigen oder cyclischen Ether, vorzugsweise Tetrahydrofuran, in situ erzeugt und diese Lösung bzw. Suspension des Alkoholates bzw. Thiolates bei Temperaturen zwischen -40°C und 30°C mit der Brommethylverbindung umgesetzt.

Zur Hydrogenolyse der Benzylester wird vorzugsweise Palladium auf Aktivkohle als Katalysator eingesetzt. Als Lösungsmittel kommen vor allem niedere Alkohole, insbesondere Methanol oder Ethanol, in Frage. Die Temperatur kann zwischen 0°C und dem Siedepunkt des betreffenden Lösungsmittels variiert werden, vorzugsweise wird bei Atmosphärendruck und einer Temperatur von 15 bis 30°C gearbeitet. Zur Überführung der gebildeten Carbonsäure in ein Carbonsäurehalogenid, insbesondere ein Carbonsäurechlorid, stehen verschiedene literaturbekannte Verfahren zur Auswahl.

Zur Herstellung der Carbonsäurechloride eignet sich besonders das aus Oxalylchlorid und Dimethylformamid gebildete Dimethylchlormethylidenammoniumchlorid (vgl. Helv. Chim. Acta 61 (1978), 1675-1681) oder Phosphorpentachlorid (vgl. Chem. Pharm. Bull. 28 (1980), 2809-2812).

Bei der Umsetzung von Carbonsäuren mit Dimethylchlormethylidenammoniumchlorid wird unter Stickstoffatmosphäre in einem inerten organischen Lösungsmittel, wie z.B. Acetonitril oder Dioxan, und bei Temperaturen zwischen -30°C und 30°C, vorzugsweise jedoch bei 0°C gearbeitet.
Die anschließende Reaktion der Carbonsäurechloride mit entsprechend substituierten Aminen erfolgt im Eintopfverfahren bei Temperaturen von 0°C bis 50°C, vorzugsweise bei 20°C.

Die Umsetzung der Carbonsäuren mit Phosphorpentachlorid wird in den in der Literatur für analoge Umsetzungen von Carbonsäuren beschriebenen Lösungsmitteln durchgeführt, vorzugsweise in chlorierten Kohlenwasserstoffen, wie Chloroform, Dichlormethan, 1,2-Dichlorethan oder Tetrachlorkohlenstoff im Temperaturbereich zwischen -20°C und 20°C.
Die Weiterreaktion zu Amiden erfolgt wie oben beschrieben.

Die vorliegende Erfindung betrifft weiterhin ein Verfahren zur Herstellung von 2-Phthalimidomethyl-1,4-dihydropyridin-Derivaten der allgemeinen Formel IV dadurch gekennzeichnet, daß man 1,4-Dihydropyridin-Derivate der allgemeinen Formel V,

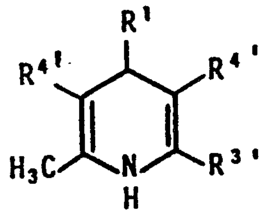

(V)

in welcher $R^1$, $R^{3'}$ und $R^{4'}$ die oben angegebene Bedeutung besitzen, an der Methylgruppe in Position 2 bromiert und die so erhaltenen Brommethylverbindungen mit Phthalimidkalium in die gewünschten 2-Phthalimidomethyl-1,4-dihydropyridine überführt.

Die für das Verfahren eingesetzten Dihydropyridine der allgemeinen Formel V sind bekannt (vgl. z.B. Chem. Rev. 82 (1982), S. 223) oder sie können in analoger Weise hergestellt werden.

Die Bromierung an der Methylgruppe in Position 2 der Dihydropyridine erfolgt mit Pyridiniumbromid-Perbromid in Gegenwart einer Base (vgl. Synthesis 1984, 617). Als Base wird Pyridin oder ein geeignetes tertiäres Amin eingesetzt. Geeignete Lösungsmittel für die Bromierung sind insbesondere chlorierte Kohlenwasserstoffe, wie Chloroform, Dichlormethan, Tetrachlorkohlenstoff oder 1,2-Dichlorethan. Die Herstellung der Brommethylverbindungen erfolgt im Temperaturbereich von -20°C bis 20°C, vorzugsweise jedoch bei 0°C. Die Reinigung der Produkte erfolgt bevorzugt durch Chromatographie.

Die weitere Umsetzung der Brommethyldihydropyridine zu Phthalimid-Derivaten der allgemeinen Formel IV erfolgt in literaturbekannter Weise (vgl. Houben-Weyl, Methoden der organischen Chemie, Bd. 11/1, 1957, S. 79ff). Als Lösungsmittel kommen polare Solventien wie Dimethylsulfoxid, Dimethylacetamid, cyclische Ether oder vorzugsweise Dimethylformamid in Frage. Die Reaktionstemperatur kann zwischen 0°C und dem jeweiligen Siedepunkt des verwendeten Lösungsmittels variiert werden, bevorzugt wird jedoch bei Raumtemperatur gearbeitet. Die Produkte werden durch Kristallisation gereinigt.

Saure oder basische Verbindungen der allgemeinen Formel I, welche für $R^4$ eine Carboxylgruppe bzw. ein basisches Zentrum an $R^3$ oder $R^4$ aufweisen, überführt man zum Zwecke der Reinigung und aus galenischen Gründen bevorzugt in kristalline, pharmakologisch verträgliche Salze.

Für den Fall, daß $R^4$ eine Carboxylgruppe darstellt, lassen sich mit Basen, wie z.B. Hydroxiden oder Carbonaten, entsprechende Salze der Alkali- oder Erdalkalimetalle herstellen. Wenn die Reste $R^3$ und/oder $R^4$ basischen Charakter aufweisen, werden Salze in üblicher Weise durch Neutralisation der Basen mit entsprechenden anorganischen oder organischen Säuren erhalten. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure oder Bernsteinsäure in Frage.

Da die erfindungsgemäßen Verbindungen der allgemeinen Formel I am C-4 ein chirales Zentrum aufweisen, können sie entweder als racemische Gemische oder in Form der Enantiomeren vorliegen.

Die Verbindungen der allgemeinen Formel I sind hochwirksame Calciumantagonisten.

Aufgrund ihrer gefäßspasmolytischen Wirkungen sind sie vor allem bei cerebralen, cardialen und peripheren Gefäßerkrankungen indiziert. Die Pyrrolo[3,4-b]pyridine der vorliegenden Erfindung sind daher wertvolle Mittel für die Bekämpfung der Herz-Kreislauf-Mortalität.

Die erfindungsgemäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z.B. Tartrat- und Citrat-Puffer, Ethanol, Komplexbildner (wie Ethylendiamin-tetraessigsäure und deren nichttoxische Salze) sowie hochmolekulare Polymere (wie flüssiges Polyethylenoxid) zur Viskositätsregulierung.

Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykol); für orale Applikation geeignete Zubereitungen können gewünschtenfalls zusätzlich Geschmacks- und/oder Süßstoffe enthalten.

Die enteral verabreichten Einzeldosen liegen im Bereich von etwa 5 bis 250 mg, vorzugsweise 20 bis 100 mg. Parenteral werden etwa 1 bis 20 mg gegeben.

Die folgenden Beispiele dienen der näheren Erläuterung der Erfindung:

Beispiel 1:

(±)-2-Brommethyl-1,4-dihydro-6-methyl-4-(2-trifluormethylphenyl)pyridin-3,5-dicarbonsäuredimethylester

14 g (38 mMol) (±)-1,4-Dihydro-2,6-dimethyl-4-(2-trifluormethylphenyl)-pyridin-3,5-dicarbonsäuredimethylester werden in 300 ml Dichlormethan gelöst und mit 4 ml (50 mMol) Pyridin versetzt. Zu dieser Lösung, die auf -5 bis 0°C gekühlt wird, werden portionsweise 14 g (44 mMol) Pyridiniumbromid-Perbromid zugegeben. Nach 40 Minuten Rühren bei -5 bis 0°C wird die Mischung im Vakuum auf 150 ml eingeengt und anschließend an Kieselgel mit Dichlormethan chromatographiert. Aus der Fraktion mit dem Rf 0,5 (Toluol/Essigester 3:1) erhält man nach Eindampfen des Lösungsmittels im Vakuum (±)-2-Brommethyl-1,4-dihydro-6-methyl-4-(2-trifluormethylphenyl)pyridin-3,5-dicarbonsäuredimethylester als gelbes Öl.
$^1$H-NMR (90 MHz; $CDCl_3$), δ (ppm): 2,31 (s; $CH_3$), 3,60 und 3,62 (2 s; 2 $CO_2CH_3$), 4,63 (s; $CH_2Br$), 5,52 (br.s; 4-H), 6,24 (br.s; NH), 7,15 - 7,80 (m; $C_6H_4$).

Analog werden folgende Verbindungen erhalten:

(±)-2-Brommethyl-1,4-dihydro-6-methyl-4-phenylpyridin-3,5-dicarbonsäuredimethylester (1.a)
Gelbes Öl
$^1$H-NMR (90 MHz; $CDCl_3$), δ (ppm): 2,32 (s; $CH_3$), 3,62 und 3,64 (2 s; 2 $CO_2CH_3$), 4,70 (q; $CH_2Br$), 5,10 (s; 4-H), 6,48 (s; NH), 7,26 - 8,22 (m; $C_6H_4$).

(±)-2-Brommethyl-1,4-dihydro-6-methyl-4-(3-nitrophenyl)pyridin-3,5-dicarbonsäuredimethylester (1.b)
Gelbes Öl
$^1$H-NMR (90 MHz; $CDCl_3$), δ (ppm): 2,33 (s; $CH_3$), 3,65 und 3,67 (2 s; 2 $CO_2CH_3$), 4,74 (q; $CH_2Br$), 5,03 (s; 4-H), 6,08 (br.s; NH), 7,25 (m; $C_6H_5$).

Beispiel 2:

(±)-1,4-Dihydro-6-methyl-2-phthalimidomethyl-4-(2-trifluormethylphenyl)-pyridin-3,5-dicarbonsäuredimethylester

9 g (20 mMol) (±)-2-Brommethyl-1,4-dihydro-6-methyl-4-(2-trifluormethylphenyl)pyridin-3,5-dicarbonsäuredimethylester gelöst in 150 ml absolutem Dimethylformamid werden zu einer Suspension von 10 g (54 mMol) Phthalimidkalium in 100 ml Dimethylformamid unter Rühren zugesetzt. Man läßt drei Stunden bei Raumtemperatur rühren, filtriert von überschüssigem Phthalimidkalium ab und engt das Filtrat im Vakuum auf ca. 50 ml ein. Es werden 300 ml Dichlormethan zugesetzt und die organische Phase dreimal mit je 300 ml Wasser ausgeschüttelt. Nach Trocknen über Natriumsulfat wird im Vakuum eingeengt. Aus Ether kristallisieren blaßgelbe Nadeln vom Schmp. 170-172°C.

In analoger Weise werden die folgenden Verbindungen erhalten:

(±)-1,4-Dihydro-6-methyl-2-phthalimidomethyl-4-(3-nitrophenyl)pyridin-3,5-dicarbonsäuredimethylester (2.a)
Schmp. 226-228°C aus Dichlormethan/Ether

(±)-1,4-Dihydro-6-methyl-4-phenyl-2-phthalimidomethylpyridin-3,5-dicarbonsäuredimethylester (2.b)
Schmp. 172°C aus Dichlormethan/Ether

**Beispiel 3:**

(±)-4,5,6,7-Tetrahydro-2-methyl-5-oxo-4-(2-trifluormethylphenyl)-1H-pyrrolo[3,4-b]pyridin-3-carbonsäuremethylester

9,7 g (19 mMol) (±)-1,4-Dihydro-6-methyl-2-phthalimido-4-(trifluormethylphenyl)pyridin-3,5-dicarbonsäuredimethylester werden in 40 ml Ethanolamin 15 Minuten auf 80°C erwärmt. Zu der erkalteten Lösung gibt man vorsichtig ca. 80 ml Wasser, saugt die ausgefallenen Kristalle ab und wäscht gründlich mit Wasser und Dichlormethan. Aus Essigester erhält man blaßgelbe Kristalle vom Schmp. 232-235°C.

In analoger Weise werden die folgenden Verbindungen erhalten:

(±)-4,5,6,7-Tetrahydro-2-methyl-5-oxo-4-phenyl-1H-pyrrolo[3,4-b]pyridin-3-carbonsäuremethylester (3.a)
Schmp. 262-263°C aus Methanol/Wasser

(±)-4,5,6,7-Tetrahydro-2-methyl-4-(3-nitrophenyl)-5-oxo-1H-pyrrolo-[3,4-b]pyridin-3-carbonsäuremethylester (3.b)
Schmp. 248-250°C aus Ethylacetat/Methanol

(±)-4,5,6,7-Tetrahydro-2-methyl-5-oxo-4-(2-trifluormethylphenyl)-1H-pyrrolo[3,4-b]pyridin-3-carbonsäure-[2-(N-benzyl-N-methylamino)ethyl]ester (3.c)
Schmp. 134-135°C aus Diisopropylether/Ethylacetat

**Beispiel 4:**

(±)-4,7-Dihydro-5-methoxy-2-methyl-4-(2-trifluormethylphenyl)-1H-pyrrolo[3,4-b]pyridin-3-carbonsäuremethylester · Hydrochlorid

3 g (8,5 mMol) (±)-4-(2-Trifluormethylphenyl)-4,5,6,7-tetrahydro-2-methyl-5-oxo-1H-pyrrolo[3,4-b]pyridin-3-carbonsäuremethylester werden in 50 ml trockenem Dichlorethan unter Eiskühlung mit 5 g (34 mMol) Trimethyloxoniumtetrafluorborat versetzt und vier Stunden unter Stickstoffatmosphäre bei dieser Temperatur gerührt. Danach wird an Kieselgel mit Dichlormethan/Methanol, $NH_3$ ges., 98:2 chromatographiert. Die Fraktion mit dem Rf 0,6 (Dichlormethan/Methanol, $NH_3$ ges., 9:1) wird im Vakuum zur Trockene eingeengt. Der ölige Rückstand wird mit einer Mischung aus 60 ml Dichlormethan und 20 ml mit Salzsäure gesättigtem Ether aufgenommen. Es kristallisiert (±)-4,7-Dihydro-5-methoxy-2-methyl-4-(2-trifluormethylphenyl)-1H-pyrrolo[3,4-b]pyridin-3-carbonsäuremethylester · Hydrochlorid in blaßgelben Nadeln vom Schmp. 147-148°C.

In analoger Weise werden folgende Verbindungen erhalten:

(±)-4,7-Dihydro-5-methoxy-2-methyl-4-phenyl-1H-pyrrolo[3,4-b]pyridin-3-carbonsäuremethylester · Hydrochlorid (4.a)
Schmp. 140-142°C aus Ether (HCl ges.)/Dichlormethan

(±)-4,7-Dihydro-5-methoxy-2-methyl-4-(3-nitrophenyl)-1H-pyrrolo-[3,4-b]pyridin-3-carbonsäuremethylester · Hydrochlorid (4.b)
Schmp. 142-143°C aus Ether (HCl ges.)/Dichlormethan

(±)-5-Ethoxy-4-(2-trifluormethylphenyl)-4,7-dihydro-2-methyl-1H-pyrrolo-[3,4-b]pyridin-3-carbonsäuremethylester · Hydrochlorid (4.c)

(±)-4,7-Dihydro-5-methoxy-2-methyl-4-(2-trifluormethylphenyl)-1H-pyrrolo-[3,4-b]pyridin-3-carbonsäureethylester · Hydrochlorid (4.d)

(±)-4,7-Dihydro-5-methoxy-2-methyl-4-(2-trifluormethylphenyl)-1H-pyrrolo-[3,4-b]pyridin-3-carbonsäurebenzylester · Hydrochlorid (4.e)
Schmp. 148-150°C aus Ether (HCl ges.)/Dichlormethan

(±)-4,7-Dihydro-5-methoxy-2-methyl-4-(2-trifluormethylphenyl)-1H-pyrrolo-[3,4-b]pyridin-3-carbonsäure-[2-(N-benzyl-N-methylamino)ethyl]ester · Dihydrochlorid (4.f)

(±)-4,7-Dihydro-5-methoxy-2-methyl-4-(2-trifluormethylphenyl)-1H-pyrrolo-[3,4-b]pyridin-3-carbonsäure · Hydrochlorid (4.g)

(±)-4-(2,3-Dichlorphenyl)-4,7-dihydro-5-methoxy-2-methyl-1H-pyrrolo[3,4-b]-pyridin-3-carbonsäureethylester · Hydrochlorid (4.h)

(±)-4-(2-Chlor-3-trifluormethylphenyl)-4,7-dihydro-5-methoxy-2-methyl-1H-pyrrolo[3,4-b]pyridin-3-carbonsäureethylester · Hydrochlorid (4.i)

(±)-4-(2-Cyanophenyl)-4,7-dihydro-5-methoxy-2-methyl-1H-pyrrolo[3,4-b]-pyridin-3-carbonsäureethylester · Hydrochlorid (4.j)

(±)-4-(2-Difluormethoxyphenyl)-4,7-dihydro-5-methoxy-2-methyl-1H-pyrrolo-[3,4-b]pyridin-3-carbonsäureethylester · Hydrochlorid (4.k)

(±)-4-(2,1,3-Benzoxadiazol-4yl)-4,7-dihydro-5-methoxy-2-methyl-1H-pyrrolo-[3,4-b]pyridin-3-carbonsäureethylester · Hydrochlorid (4.l)

(±)-4,7-Dihydro-5-methoxy-2-methyl-4-(2-thienyl)-1H-pyrrolo[3,4-b]pyridin-3-carbonsäureethylester · Hydrochlorid (4.m)

(±)-4,7-Dihydro-5-methoxy-2-methyl-4-(2-pyridyl)-1H-pyrrolo[3,4-b]pyridin-3-carbonsäureethylester · Hydrochlorid (4.n)

(±)-4,7-Dihydro-5-methoxy-2-methyl-4-(2-trifluormethylphenyl)-1H-pyrrolo-[3,4-b]pyridin-3-carbonsäureamid · Hydrochlorid (4.o)

(±)-4,7-Dihydro-5-methoxy-2-methyl-4-(2-trifluormethylphenyl)-1H-pyrrolo-[3,4-b]pyridin-3-carbonsäure-N-ethylamid · Hydrochlorid (4.p)

(±)-4,7-Dihydro-5-methoxy-2-methyl)-4-(2-trifluormethylphenyl)-1H-pyrrolo-[3,4-b]pyridin-3-carbonsäure-[2-(N-benzyl-N-methylamino)ethyl]amid · Hydrochlorid (4.q)

(±)-2-(2-Aminoethoxymethyl)-4,7-dihydro-5-methoxy-4-(2-trifluormethylphenyl)-1H-pyrrolo[3,4-b]pyridin-3-carbonsäureethylester · Hydrochlorid (4.r)

(±)-2-(2-Aminoethylthiomethyl)-4,7-dihydro-5-methoxy-4-(2-trifluormethylphenyl)-1H-pyrrolo[3,4-b]pyridin-3-carbonsäureethylester · Hydrochlorid (4.s)

Patentansprüche für die Vertragsstaaten BE, CH, DE, FR, GB, IT, LI, LU, NL, SE

1) Pyrrolo[3,4-b]pyridin-Derivate der allgemeinen Formel I

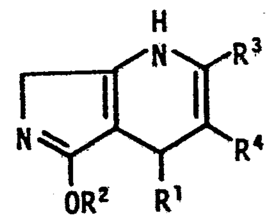

(I)

bzw. für den Fall, daß $R^2$ Wasserstoff bedeutet, deren tautomere Form der allgemeinen Formel Ia

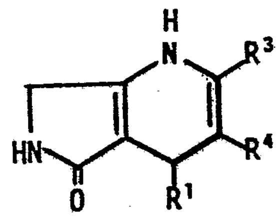

(Ia)

in welchen

$R^1$ einen unsubstituierten oder substituierten aromatischen oder heteroaromatischen Ring oder ein kondensiertes aromatisches oder heterocyclisches Ringsystem

$R^2$ Wasserstoff oder eine Methyl- oder Ethylgruppe

$R^3$ einen Kohlenwasserstoffrest mit bis zu 3 Kohlenstoffatomen der auch Sauerstoff-, Schwefel- oder Stickstoffatome enthalten kann,

$R^4$ eine Carboxylgruppe, einen Alkoxycarbonyl- oder einen Carboxamidrest mit bis zu 11 Kohlenstoffatomen, der gegebenenfalls auch Sauerstoff- oder Stickstoffatome enthalten kann, bedeutet,

sowie gegebenenfalls deren pharmakologisch unbedenkliche Salze.

2) Pyrrolo[3,4-b]pyridin-Derivate der allgemeinen Formel I, gemäß Anspruch 1 in welcher

$R^1$ einen unsubstituierten oder durch Halogen, Cyano, Nitro, Difluormethoxy oder Trifluormethyl mono- oder disubstituierten Phenylrest, einen Naphthylrest oder einen 2-Pyridyl-, 2-Thienyl- oder 2,1,3-Benzoxadiazolylrest,

$R^2$ eine Methylgruppe,

$R^3$ eine Methyl-, Aminoethoxymethyl- oder Aminoethylthiomethylgruppe, und

$R^4$ einen Carboxyl- oder einen Alkoxycarbonylrest der allgemeinen Formel II

$$-CO_2R^5 \qquad (II)$$

in welcher $R^5$ entweder Wasserstoff, eine Methyl-, Ethyl- , Benzyl-, Aminoalkyl- oder N-Benzyl-N-Methylaminoethylgruppe, oder $R^4$ einen Carboxamidrest der allgemeinen Formel III,

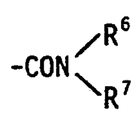

(III)

bedeutet worin $R^6$ und $R^7$ gleich oder verschieden sein können und Wasserstoff, eine Ethyl- oder N-Benzyl-N-methylaminoethylgruppe bedeuten.

3) Verfahren zur Herstellung von Pyrrolo-[3,4-b]pyridin-Derivaten der allgemeinen Formeln I bzw. Ia, gemäß der Ansprüche 1 und 2, dadurch gekennzeichnet, daß man entweder

a) 2-Phthalimidomethyl-1,4-dihydropyridin-Derivate der allgemeinen Formel IV

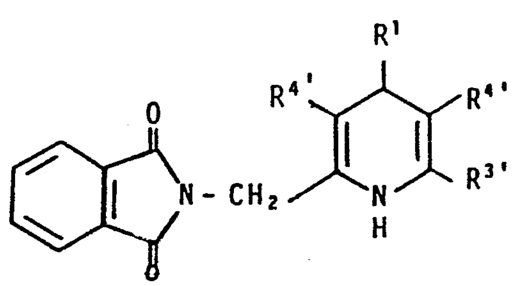

(IV)

in welcher

$R^1$ einen unsubstituierten oder substituierten aromatischen oder heteroaromatischen Ring oder ein kondensiertes aromatisches oder heterocyclisches Ringsystem,

$R^{3'}$ eine Methylgruppe,

$R^{4'}$ einen Carboxyl- oder einen Alkoxycarbonylrest der allgemeinen Formel II bedeutet,

mit einem Aminoalkohol durch Ringschluß in die Pyrrolo[3,4-b]pyridin-Derivate der allgemeinen Formel Ia überführt und anschließend in an sich bekannter Weise am Lactamsauerstoffatom, alkyliert, oder

b) Verbindungen der allgemeinen Formel I, bei denen $R^3$ für eine Methylgruppe steht, an dieser bromiert und mit entsprechend substituierten Alkoholen oder Thiolen umsetzt zu Verbindungen der allgemeinen Formel I, bei denen $R^3$ eine Aminoethoxymethyl- oder Aminoethylthiomethylgruppe bedeutet, oder

c) Verbindungen der allgemeinen Formel I, bei denen $R^4$ einen Benzyloxycarbonylrest bedeutet, in an sich bekannter Weise hydrogenolytisch zur entsprechenden Carbonsäure spaltet und diese wiederum in an sich bekannter Weise über die Zwischenstufe eines Säurehalogenides mit entsprechend substituierten Aminen umsetzt zu Verbindungen der allgemeinen Formel I, bei denen $R^4$ für eine Carboxylgruppe oder eine substituierte oder unsubstituierte Carboxamidgruppe steht.

4.) Arzneimittel, dadurch gekennzeichnet, daß es neben den üblichen Hilfs- und Zusatzstoffen mindestens eine Verbindung gemäß der Ansprüche 1 oder 2 enthält.

5.) Verfahren zur Verwendung von Verbindungen entsprechend der Ansprüche 1 und 2 zur Herstellung von Arzneimitteln zur Behandlung von Gefäßerkrankungen.

0234517

For the Contracting State(s)
Für den (die) Vertragsstaat(en)
Pour l' (les) Etat(s) contractant(s)

AT ES GR

Patentansprüche für die Vertragsstaaten AT, ES, GR

1) Verfahren zur Herstellung der Pyrrolo[3,4-b]pyridin-Derivate der allgemeinen Formel I

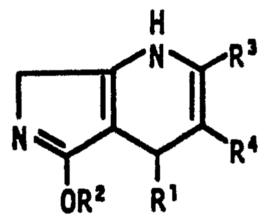

(I)

bzw. für den Fall, daß $R^2$ Wasserstoff bedeutet, deren tautomere Form der allgemeinen Formel Ia

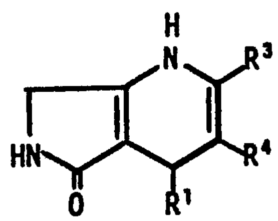

(Ia)

in welchen

$R^1$ einen unsubstituierten oder substituierten aromatischen oder heteroaromatischen Ring oder ein kondensiertes aromatisches oder heterocyclisches Ringsystem

$R^2$ Wasserstoff oder eine Methyl- oder Ethylgruppe

$R^3$ einen Kohlenwasserstoffrest mit bis zu 3 Kohlenstoffatomen der auch Sauerstoff-, Schwefel- oder Stickstoffatome enthalten kann,

$R^4$ eine Carboxylgruppe, einen Alkoxycarbonyl- oder einen Carboxamidrest mit bis zu 11 Kohlenstoffatomen, der gegebenenfalls auch Sauerstoff- oder Stickstoffatome enthalten kann, bedeutet,

sowie gegebenenfalls deren pharmakologisch unbedenkliche Salze,

For the Contracting State(s)
Für den (die) Vertragsstaat(en)
Pour l' (les) Etat(s) contractant(s)
0234517
AT ES GR

dadurch gekennzeichnet, daß man entweder

a) 2-Phthalimidomethyl-1,4-dihydropyridin-Derivate der allgemeinen Formel IV

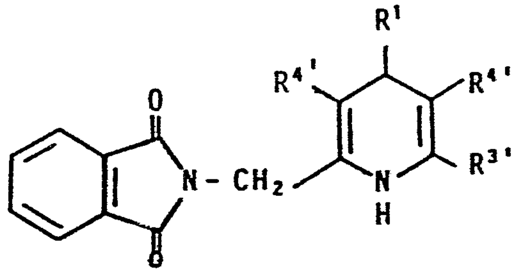

(IV)

in welcher

$R^1$ einen unsubstituierten oder substituierten aromatischen oder heteroaromatischen Ring oder ein kondensiertes aromatisches oder heterocyclisches Ringsystem,

$R^{3'}$ eine Methylgruppe,

$R^{4'}$ einen Carboxyl- oder einen Alkoxycarbonylrest der allgemeinen Formel II bedeutet,

mit einem Aminoalkohol durch Ringschluß in die Pyrrolo[3,4-b]pyridin-Derivate der allgemeinen Formel Ia überführt und anschließend in an sich bekannter Weise am Lactamsauerstoffatom, alkyliert, oder

b) Verbindungen der allgemeinen Formel I, bei denen $R^3$ für eine Methylgruppe steht, an dieser bromiert und mit entsprechend substituierten Alkoholen oder Thiolen umsetzt zu Verbindungen der allgemeinen Formel I, bei denen $R^3$ eine Aminoethoxymethyl- oder Aminoethylthiomethylgruppe bedeutet, oder

c) Verbindungen der allgemeinen Formel I, bei denen $R^4$ einen Benzyloxycarbonylrest bedeutet, in an sich bekannter Weise hydrogenolytisch zur entsprechenden Carbonsäure spaltet und diese wiederum in an sich bekannter Weise über die Zwischenstufe eines Säurehalogenides mit entsprechend substituierten Aminen umsetzt zu Verbindungen der allgemeinen Formel I, bei denen $R^4$ für eine Carboxylgruppe oder eine substituierte oder unsubstituierte Carboxamidgruppe steht.

2) Verfahren zur Herstellung der Pyrrolo[3,4-b]pyridin-Derivate der allgemeinen Formel I, gemäß Anspruch 1, in welcher

$R^1$ einen unsubstituierten oder durch Halogen, Cyano, Nitro, Difluormethoxy oder Trifluormethyl mono- oder disubstituierten Phenylrest, einen Naphthylrest oder einen 2-Pyridyl-, 2-Thienyl- oder 2,1,3-Benzoxadiazolylrest,

$R^2$ eine Methylgruppe,

$R^3$ eine Methyl-, Aminoethoxymethyl- oder Aminoethylthiomethylgruppe, und

$R^4$ einen Carboxyl- oder einen Alkoxycarbonylrest der allgemeinen Formel II

$$-CO_2R^5 \qquad (II)$$

in welcher $R^5$ entweder Wasserstoff, eine Methyl-, Ethyl- , Benzyl-, Aminoalkyl- oder N-Benzyl-N-Methylaminoethylgruppe, oder $R^4$ einen Carboxamidrest der allgemeinen Formel III,

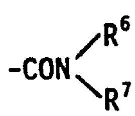 (III)

bedeutet worin $R^6$ und $R^7$ gleich oder verschieden sein können und Wasserstoff, eine Ethyl- oder N-Benzyl-N-methylaminoethylgruppe bedeuten.

3.) Verfahren zur Verwendung von Verbindungen entsprechend der Ansprüche 1 und 2 zur Herstellung von Arzneimitteln zur Behandlung von Gefäßerkrankungen.

For the Contracting State(s)
Für den (die) Vertragsstaat(en)
Pour l' (les) Etat(s) contractant(s)

AT ES GR

**0234517**

Europäisches Patentamt

# EUROPÄISCHER RECHERCHENBERICHT

Nummer der Anmeldung

EP 87 10 2399

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A | EP-A-0 133 530 (GÖDECKE)<br>* Patentansprüche 1,4 *<br>----- | 1,4 | C 07 D 471/04<br>A 61 K 31/435//<br>(C 07 D 471/04<br>C 07 D 221:00<br>C 07 D 209:00 ) |

| RECHERCHIERTE SACHGEBIETE (Int. Cl.4) |
|---|
| C 07 D 471/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 25-05-1987 | ALFARO I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82